(19) Europäisches Patentamt
European Patent Office
Office européen des brevets

(11) **EP 3 069 763 A1**

(12) **EUROPEAN PATENT APPLICATION**

(43) Date of publication:
21.09.2016 Bulletin 2016/38

(51) Int Cl.:
*A61Q 17/04* (2006.01)    *A61K 8/97* (2006.01)
*A61K 8/49* (2006.01)    *A61K 8/67* (2006.01)
*A61Q 19/08* (2006.01)

(21) Application number: 15000770.6

(22) Date of filing: 16.03.2015

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(71) Applicant: **The Boots Company PLC**
**Nottingham NG90 1BS (GB)**

(72) Inventor: **BELL, Michael David**
**Derbyshire DE55 6EL (GB)**

(74) Representative: **Goodier, Claire-Louise et al**
**The Boots Company PLC**
**Group IP/Patents - D90 West F20**
**1 Thane Road West**
**Nottingham NG90 1BS (GB)**

(54) **Topical cosmetic compositionS against free radicals**

(57)    According to the present invention there is provided a cosmetic liquid composition comprising an antioxidant complex, said complex comprising at least one antioxidant polyphenolic agent, at least one vitamin E or a vitamin E derivative; and a chromane antioxidant.

Figure 2

**Description**

Technical Field

The present invention relates to a cosmetic composition comprising an improved antioxidant complex providing enhanced protection for the skin against environmental insults, such as UVA or UVB, infra-red and visible light, smoke and/or pollution.

Background to the Invention

The skin is designed specifically to protect from the environment. Thus skin is under constant attack from exogenous environmental insults, such as UVA or UVB, infra-red and visible light, cigarette smoke and/or pollution. Such environmental insults either directly or indirectly result in skin cell damage, caused by the generation of reactive species and free radicals, such as superoxide anions, hydrogen peroxide, hydroxyl ions, peroxyl ions, ozone, singlet oxygen, sulphur oxide, nitrogen oxide, carbon monoxide, alkoxyl ion, peroxynitrite and heavy metals. Reactive oxygen species (ROS), reactive carbonyl species (RCS) and reactive nitrogen species (RNS) need to be particularly considered. The above reactive species are known to be harmful to skin, causing damage to biological macromolecules, oxidising proteins, DNA and lipids. If the skin is left unguarded the resulting oxidative damage results in the increased appearance of lines and wrinkles, actinic lentigines, dyspigmentation, rough skin, actinic telangiectasia loss of elasticity and firmness and sagging, all understood generally as signs of ageing.

To prevent sunlight mediated damage of skin cells and associated damage due to sunlight initiating the formation of free radicals in the skin, compositions containing a sunscreen may be used. These compositions generally contain an inorganic sunscreen such as titanium dioxide which reflects the sun's rays, or one or more of an organic sunscreen which absorbs the rays.

A further measure to protect the skin is to use compositions containing antioxidants which act as free radical quenchers. The quenchers react with the free radicals, terminating the chain reaction that free radicals customarily propagate which so damage the skin. The Applicants prior patent EP1217983 describes compositions suitable for topical application to the skin comprising three antioxidant species selected from a defined group, and in some cases a sunscreen material. EP2197413 also describes compositions suitable for topical application to the skin comprising ginkgo extract, emblica extract and a further antioxidants selected from the group consisting of dimethyl methoxy chromanol, pine bark and rosemary extract.

There is however a continuing desire amongst consumers and cosmetic manufacturers to further improve skin care products, increasing and improving the benefits that can be delivered through application of a cosmetic composition. The skincare compositions of the present invention are shown to protect the skin more effectively from exposure to exogenous environmental insults. Thus the present invention relates not only to skincare compositions, but also to methods of providing improved protection against damage to skin caused by exposure to exogenous factors such as sunlight, environmental and/or atmospheric pollution.

Summary of the invention

According to the present invention there is provided a cosmetic liquid composition comprising an antioxidant complex, said complex comprising at least one antioxidant plant polyphenolic agent, at least one vitamin E or a vitamin E derivative, and a chromane antioxidant.

According to the present invention there is also provided a method of treating skin by the application of the composition described herein.

Detailed Description of the Invention

The composition of the present invention comprises a superior and synergistic antioxidant complex comprising at least three separate components, namely an antioxidant plant polyphenolic agent, vitamin E or derivative thereof, and a chromane antioxidant. The method of action of antioxidants is generally not well understood. However, the applicants have found that the specific combination of antioxidant agents of the present invention provides improved protection for the skin against a wide variety of reactive species, specifically including those responsible for oxidative damage to biological macromolecules, proteins, DNA and lipids. Furthermore, the applicants have found that the present combination of antioxidant agents provide synergistic benefits in protecting the skin against oxidative stress. Although not wishing to be bound by theory, it is believed that the components of the present complex provide antioxidant capability with varying levels of efficiency and kinetics, which deliver longer lasting antioxidant effect and regeneration of antioxidant species.

Composition

The compositions of the present invention may be water, micro-emulsion or emulsion based. Where the compositions are emulsions, they may be water-in-oil or oil-in-water emulsion. Where in the form of a water-in-oil emulsion, water is present at a level of from 20% to 60%, more preferably from 20% to 50%. Most preferably water in a water-in-oil emulsion is present in a range of from 35% to 45% of the emulsion composition.
Alternatively, and most preferably the composition is an oil- in-water emulsion. Said composition comprises from 40% to 95%, more preferably from 60% to 95% by weight of the composition of water.
The oil phase of the emulsion can be provided by any suitable oily component. Suitable oils for the oil phase may comprise for example: a) hydrocarbon oils, such as paraffin or mineral oils; b) waxes, such as beeswax or paraffin wax; c) natural oils, such as sunflower oil, apricot kernel oil, shea butter or jojoba oil; d) silicone oils, such as dimethicone, silicone elastomer, cyclomethicone or cetylidimethicone; e) fatty acid esters and ethers, such as isopropyl palmitate or isopropyl myristate and polypropylene glycol-15 stearyl ether; f) fatty alcohols, such as cetyl alcohol or stearyl alcohol; or g) mixtures thereof, for example, the blend of waxes available commercially under the trade name Cutina (BASF). Preferably, the emulsion comprises 0.1% to 55%, more preferably from 15% to 50%, most preferably from 30% to 45% by weight of the emulsion, of oil phase. Preferably the oil phase of the emulsion comprises oil at a level between 50% and 99.9% by weight of the oil phase. More preferably the oil phase comprises oil at a level of from 60% to 99.9%, more preferably from 70% to 99.9%, and even more preferably from 80% to 99.9% by weight of the oil phase.
Preferably the oil phase of the water-in-oil emulsion comprises silicone. Where present, the silicone-containing oil phase preferably comprises an organo polysiloxane oil. The organopolysiloxane oil for use in the composition may be volatile, non-volatile, or a mixture of volatile and non-volatile silicones. The term "nonvolatile" as used in this context refers to those silicones that are liquid or gel under ambient conditions and have a flash point (under one atmospheric of pressure) of greater than 100°C. The term "volatile" as used in this context refers to all other silicone oils. Suitable organopolysiloxanes can be selected from a wide variety of silicones spanning a broad range of volatilities and viscosities. Examples of suitable organopolysiloxane oils include polyalkylsiloxanes, cyclic polyalkylsiloxanes, polyalkylarylsiloxanes, trimethylsiloxysilicate, dimethiconols, silicone elastomer and mixtures thereof.
Preferred for use herein are organopolysiloxanes selected from polyalkylsiloxanes, alkyl substituted dimethicones, cyclomethicones, trimethylsiloxysilicates, dimethiconols, polyalkylaryl siloxanes, and mixtures thereof. More preferred for use herein are polyalkylsiloxanes and cyclomethicones. Preferred among the polyalkylsiloxanes are dimethicones.
The compositions of the present invention may include an emulsifying crosslinked organopolysiloxane elastomer, a non-emulsifying crosslinked organopolysiloxane elastomer, or a mixture thereof. The term "non-emulsifying," as used herein, defines crosslinked organopolysiloxane elastomers from which polyoxyalkylene units are absent. The term "emulsifying," as used herein, means crosslinked organopolysiloxane elastomers having at least one polyoxyalkylene (e.g., polyoxyethylene or polyoxypropylene) unit. Preferred emulsifying elastomers herein include polyoxyalkylene modified elastomers formed from divinyl compounds, particularly siloxane polymers with at least two free vinyl groups, reacting with Si-H linkages on a polysiloxane backbone. Preferably, the elastomers are dimethyl polysiloxanes crosslinked by Si-H sites on a molecularly spherical MQ resin. Emulsifying crosslinked organopolysiloxane elastomers can notably be chosen from the crosslinked polymers described in U.S. Pat. Nos. 5,412,004; 5,837,793 and 5,811,487. In addition, an emulsifying elastomer comprised of dimethicone copolyol crosspolymer (and) dimethicone is available from Shin Etsu under the trade name KSG-21.
Advantageously, the non-emulsifying elastomers are dimethicone crosspolymers. Such dimethicone crosspolymers are supplied by a variety of suppliers including Dow Corning (EL9240). Other dimethicones crosspolymer are available from General Electric (SFE 839), Shin Etsu (KSG-15, 16, 18 [dimethicone/phenyl vinyl dimethicone crosspolymer]), and Grant Industries (GRANSIL(TM) line of elastomers). Cross-linked organopolysiloxane elastomers useful in the present invention and processes for making them are further described in US4970252, US5760116 and US5654362. Commercially available elastomers preferred for use herein are Dow Coming's 9040 silicone elastomer blend, Shin Etsu's KSG-21, and mixtures thereof.
Where present preferably the oil phase comprises silicone, and most preferably, a silicone elastomer. Preferably, the composition includes from 20% to 35%, by weight of the composition, of the silicone elastomer raw material.

Antioxidant Polyphenolic Agent

The composition of the present invention comprises at least one antioxidant polyphenolic agent. By the term antioxidant plant polyphenolic agent we mean a plant extract, or derivative thereof, comprising flavonoid species, including flavones, flavonols, flavanones, flavanols anthrocyanidins and isoflavonoids; phenolic acid species; stilbenes; lignans and mixtures thereof, which provide an antioxidant benefit. Antioxidant benefit is measured using the total antioxidant capacity (TAC) assay described herein. Plants provide a rich and cheap source of polyphenolic agents, and are therefore an efficient source of said antioxidants. Similar actives can be prepared synthetically and as such are analogues of said plant polyphenolic agents. Such analogs however represent an inefficient source of said active materials and as such are not preferred.

Antioxidant polyphenolic agents are preferably extracts from plants selected form the groups consisting of Mulberry (e.g. morus alba), Ginseng (e.g. Panax ginseng), Raspberry, Oregano (e.g. origanum vulgare), Green tea (e.g. green leaves of camellia sinensis), White tea (e.g. camellia sinensis), Blueberry extract (e.g. vaccinium cyanococcus), French maritime pine bark (e.g. pinus pinaster, sold under the tradename Pycnogenol), Rosemary (e.g. rosmarinus officialis), Grape, including grape seed (e.g. vitis vinifera), Fennel (e.g. foeniculi fructus), Caragana sinica, Marjoram (e.g. origanum majorana), Crocus (e.g. crocus sativus), Apple (e.g. malus domestica), Coffee, Green coffee, Cherry (e.g. prunus avium), Snow algae (e.g. chlamydomonas nivalis), Emblica (e.g. Phyllanthus emblica), Gingko (e.g. Gingko biloba), Moringa (e.g. moringa oleilera), Ginger (e.g. zingiberaceae), Magnolia (e.g. magnolioideae virginiana), French saffron, Edelweiss (e.g. leontopodium alpinium), White lotus (e.g. nymphaea alba), Turmeric root, Marshmallow (e.g. althaea officianlis), Burdock (e.g. arctium lappa), Bilberry (e.g. vaccinium myrtillus), Cranberry (e.g. vaccinium oxycoccus), Pomegranate nectar (e.g. Punica granatum), Sage (e.g. salvia officinalis), Thyme (e.g. thymus vulgaris), Sunflower (e.g. helianthus annuus), wild carrot (e.g. daucus carota), Hop (e.g. humulus lupulus), Witch Hazel (e.g. hamamelis), Oak (e.g. Quercus), Camellia (e.g. theacea), Red clover (e.g. tritolium pratense), Flax (e.g. linium usitatissimum), lemon (e.g. citrus limon), birch (e.g. betula), cornflower, (e.g. centaurea cyanus), geranium, polygonum, soy (e.g. glycine max), and mixtures thereof.

Preferably said antioxidant polypenolic agent is an extract from a plant selected from the group consisting of mulberry, ginseng, grape, oregano, grape, sage, sunflower, maritime pine bark, rosemary, marjoram, crocus, french saffron, wild carrot, hop, coffee ,green coffee, witch hazel, oak, camellia, red clover, flax, ginger, magnolia, edelweiss, burdock and mixtures thereof.

Active polyphenolic species sourced from the above list of plants include those selected from the group consisting of apigenin, luteolin, quercetin, kaempferol, naringenin, hesperetin, catechin, gallocatechin, cyaniding, pelargonidin, daidzein, genistein, caffeic acid, chlorogenic acid, romsmarinic acid, gallic acid, resveratrol, ferulic acid, epigallocatechin gallate, piceatannol, secoisolariciresinol, isotaxiresinol, Miyabenol c, Luteolin and mixtures thereof.

The amounts of antioxidant plant polyphenolic agents used in the present invention are expressed as dry weights of the extract, as understood by a man skilled in the art. Preferably the antioxidant plant polyphenolic agent (plant extract) is present at 0.005% to 10% by weight, preferably 0.01% to 7%, most preferably 0.01% to 5%polyphenolic agent by weight of the composition.

Vitamin E or Vitamin E derivative

The compositions of the present invention comprise a vitamin E or vitamin E derivative. In a preferred embodiment of the present invention, the composition comprises a vitamin E or vitamin E derivative is selected from the group consisting of alpha tocopherol, beta tocopherol, gamma tocopherol, delta tocopherol, epsilon tocopherol, zeta tocopherol, eta tocopherol, tocopheryl acetate, tocopheryl succinate, tocopheryl benzoate, tocopheryl propionate, tocopheryl sorbate, tocopheryl oleate, tocopheryl orotate, tocopheryl linoleate, tocopheryl nicotinate, 2-ethylhexanoate ester and mixtures thereof.

Preferably the vitamin E or derivative thereof is present at a level of from 0.01 % to 98 %, more preferably from 0.01% to 5%, most preferably from 0.01 to 2% by weight of the composition.

Chromane Antioxidant

The composition of the present invention comprises a chromane antioxidant. Preferably said chromane antioxidant is selected from the group consisting of: methyl, di, tri and tetra C1-C6 alkyl, C1-C6 alkoxy chromanol; pentamethyl chromanol, methyl, di, tri and tetra C1-C6 alkyl, C1-C6 alkoxy chromanyl C14-C20 ester and mixtures thereof.

Preferably the chromane antioxidant is selected from the group consisting of dimethyl methoxy chromanol, tetramethyl methoxy chromanol, pentamethyl chromanol, dimethyl methoxy chomanyl palmitate, dialkyl methoxy chomanyl myristate, dimethyl methoxy chromanyl stearate, dimethyl methoxy chomanyl oleate, dimethyl methoxy chomanyl linoleate and mixtures thereof.

More preferably the chromane antioxidant is dimethyl methoxy chromanol. Said chromanol antioxidant is available under the tradename Lipochroman 6 as sold by Lipotec. Preferably the chromanol antioxidant is present at a level of from 0.0001% to 10%, more preferably from 0.001% to 7% and most preferably from 0.001% to 5% by weight of the composition.

Other Vitamins and Provitamins

The compositions of the present invention may comprise one or more additional vitamins. Preferably said compositions may comprise vitamin C, vitamin C derivatives, vitamin B, vitamin B derivatives, vitamin B1 to vitamin B12 and theirs derivatives, vitamin K, vitamin K derivatives, vitamin H, vitamin D, vitamin D derivatives, and provitamins thereof, such as panthenol and mixtures thereof. The vitamin compounds may be included as the substantially pure material, or as an extract obtained by suitable physical and/or chemical isolation from natural (e. g., plant) sources. In a preferred embodiment the compositions additionally comprise Vitamin C or derivatives thereof. More preferably the compositions comprise vitamin C or vitamin C derivatives selected from the group consisting of ascorbic acid, ascorbyl glucoside, ascorbyl palmitate, magnesium ascorbyl phosphate, sodium ascorbyl phosphate and mixtures thereof.

In another preferred embodiment the compositions comprise vitamin B3 and/or derivatives thereof. In a particularly preferred embodiment the composition comprises a vitamin B3 compounds selected from the group consisting of niacinamide, tocopherol niacotinate and mixtures thereof. In one embodiment, when vitamin compounds are present in the compositions of the instant invention, the compositions comprise from about 0.0001% to 50%, more preferably from 0.001% to 10%, still more preferably from 0.01% to 8%, and still more preferably from 0.1% to 5%, by weight of the composition, of the vitamin compound.

Emulsifiers

The present composition may comprise an emulsifier. In a preferred embodiment, the composition comprises from 0.1% to 10% emulsifier, more preferably from 0.25% to 7.5%, still more preferably from 0.5% to 5%, emulsifier by weight of the composition. The emulsifier helps disperse and suspend an aqueous water phase within a oil phase or vice versa. Suitable emulsifiers include all those suitable for the purpose and known by those skilled in the art for use in skin care products. Preferably these emulsifiers have an HLB value of or less than 14, more preferably from 2 to 14, and still more preferably from 4 to 14.

A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and chains comprising moieties derived from both ethylene oxide and propylene oxide. Other examples include alkyl-modified dimethicone copolyols, i. e., compounds which comprise C2-C30 pendant side chains. Still other useful dimethicone copolyols include materials having various cationic, anionic, amphoteric and zwitterionic pendant moieties.

More preferably, emulsifiers include various non-ionic and anionic emulsifying agents such as sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcools, alkoxylated ethers of C1-C30 fatty alcohols, polyglyceryl esters of C1-C30 fatty acids, C1-C30 esters of polyols, C1-C30 ethers of polyols, alkyl phosphates, polyoxyalkylene fatty ether phosphates, fatty acid amides, acyl lactylates, soaps, and mixtures thereof. Nonlimiting preferred examples of these non-silicon-comprising emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, steareth-20, ceteareth-20, PPG-2 methyl glucose ether distearate, ceteth-10, diethanolamine cetyl phosphate, glyceryl stearate, PEG-100 stearate, and mixtures thereof.

Peptides

The compositions of the present invention preferably comprise a peptide. Peptides are defined as compounds comprising an uninterrupted sequence of amino acids. A dipeptide comprises an uninterrupted sequence of two amino acids. Amino acids, as employed herein, include and encompass all of the naturally occurring amino acids, either in D or L configuration. Amino acids are commonly indicated with reference to the conventional three letter code and the sequence is read from left to right. The composition of the present invention preferably comprises a dipeptide selected from the group consisting of acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17, dipeptide 18, dipeptide 19, dipeptide 2, dipeptide 20, dipeptide 3, dipeptide 4, dipeptide 5, dipeptide 6, dipeptide 7, dipeptide 8, dipeptide 8 HCL, dipeptide 9, hexanoyl dipeptide 3 norleucine acetate, methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide17, palmitoyl dipeptide 5 diaminobutyroyl hydroxythreonine, palmitoyl dipeptide 5 diaminohydroxybutyrate, palmitoyl dipeptide 7 and mixtures thereof.

More preferably, the composition of the present invention comprise a dipeptide wherein said amino acid sequences of said dipeptide are selected from the group consisting of Tyr-Arg, Tyr-Val, Ala-Glu, Val-Trp, Asn-Phe, Asp-Phe and mixtures thereof. More preferably said dipeptide is selected from the group consisting of Trp-Val (tryptophan-valine), Ala-Glu (alanine-glutamine), Tyr-Arg peptide (tyrosine-argenine) and mixtures thereof. Most preferably said dipeptide is N-Acetyl Tyr-Arg -1 cetyl ester.

Dipeptides are preferably incorporated into the composition of the present invention at a level of from 0.1 to 50000ppm, more preferably from 1 to 5000 ppm, most preferably from 10 to 500ppm.

In a preferred embodiment of the present invention, the composition of the present invention also comprises additional peptides. Preferably said additional peptides are selected from the group consisting of tripeptides, tetrapeptides, pentapeptides and mixtures thereof. By tripeptides, it is meant compound comprising an uninterrupted sequence of three amino acids. By tetrapeptides, it is meant a compound comprising an uninterrupted sequence of four amino acids. By pentapeptide it is meant a compound comprising an uninterrupted sequence of five amino acids.

Tripeptides:

The compositions of the present invention preferably comprise a tripeptide. Said tripeptide may be naturally occurring or of synthetic origin. Suitable tripeptides include tripeptide 1, 2, 3, 4, 5, 6, 7, 8, 9, 10, 11, 12, 13, 14, 15, 16, 17, 18, 19, 20, 21, 22, 23, 24, 25, 26, 27, 28 ,29, 30, 31, 32, 33, 34, 35, 36, 37, 38, 39, 40, 41, 42, 43, 44, 45, 46, derivatives thereof and mixtures thereof.

Particularly preferred tripeptides comprise one or more His-based tripeptides. However another suitable tripeptide may be Arg-Lys-Arg. Particularly preferred tripeptides are based on the structure Gly-His-Lys and its analogs and derivatives thereof. These are collectively known herein as GHK-tripeptides. Indeed, the preferred tripeptide in accordance with this aspect of the invention has this exact sequence of amino acids. Analogs of the preferred tripeptide useful herein include those in which one or more of the three amino acids are reorganized or rearranged within the sequence (e.g., Gly-Lys-His) and/or where no more than two amino acids are substituted (e.g., His-Ala-Orn). However, most preferably, amino acids substituted for Gly include an aliphatic side chain such as, without limitation, beta-Ala, Ala, Val, Leu, Pro, Sarcosine (Sar) and Ile. Most preferred are Ala, Leu and Ile. The most preferable amino acid substituted for Lys or His include those having a side chain that includes, predominantly, a charged nitrogen at a pH of 6, such as, without limitation, Pro, Lys, Arg, His, Desmosine and Isodesmosine. Most preferably, Lys is replaced with Orn, Arg, or Citrulline.

Derivatives are also considered to be encompassed by the term GHK-tripeptides in accordance with the present invention, (and therefore also the more generic term tripeptides). Derivatives of GHK-tripeptides in accordance with the present invention include derivatives of the substituted and rearranged tripeptides described herein. These derivatives include, inter alia, acyl-derivatives, which are tripeptides substituted with one or more straight-chain or branched-chain, long or short chain, saturated or unsaturated, substituted with a hydroxy, amino, acyl amino, sulfate or sulfide group, or unsubstituted, which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur compriseing groups such as, without limitation $SO_3H$, SH or S-S.

His-based tripeptides include at least one Histadine amine acid. The other two amino acids in the sequence may be the same or different. Thus, contemplated are, without limitation, His-Xaa-Xaa, His-Xaa-Xbb, His-Xbb-Xaa, Xbb-His-Xbb, Xbb-His-Xaa, Xaa-His-Xbb, Xaa-Xaa-His, Xaa-Xbb-His, Xbb-Xaa-His and Xbb-Xbb-His, where Xaa and Xbb are two different amino acids, although either can be His. Preferably, at least one of the other amino acids is Gly, beta-Ala, Ala, Val, Leu, Pro, Sarcosine (Sar) or Ile. Preferably, at least one of the other amino acids is Pro, Lys, Arg, His, Desmosine and Isodesmosine. Most preferably, Lys is replaced with Orn, Arg, or Citrulline. Derivatives are also considered to be encompassed by the term His-based tripeptides in accordance with the present invention, (and therefore also the more generic term tripeptides). These derivatives include, inter alia, acyl-derivatives, which are tripeptides substituted with one or more straight-chain or branched-chain, long or short chain, saturated or unsaturated substituted or unsubstituted acyl group(s) having from 1 to 29 carbon atoms. The acyl groups which can be used are the same as those described for the GHK-tripeptides.

Particularly preferred embodiments of tripeptides in accordance with the present invention include N-Acyl-Gly-His-Lys and most preferably, N-Palmitoyl-Gly-His-Lys. Preferred commercially available tripeptide and tripeptide derivative compriseing compositions include Biopeptide-CL from SEDERMA, Maxilip(R) from SEDERMA, Biobustyl(R) from SEDERMA. The tripeptides of the present invention are preferably used in amounts that can be as little as 0.10ppm to 10,000ppm, preferably between 0.50ppm to 5,000ppm, more preferably from 1ppm to 1000ppm, and most preferably from 1ppm to 500ppm. These are again based on a % w/w basis. Thus 100,000ppm is 10% by weight of the composition.

Tetrapeptides:

The compositions of the present invention preferably comprise a tetrapeptide. These may be one or more rigin-based tetrapeptides, one or more ALAMCAT-tetrapeptides or mixtures thereof. These tetrapeptides may be naturally occurring or of synthetic origin. Suitable tetrapeptides for use in the present composition include those selected from the group consisting of tetrapeptide 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 34, 35, derivatives thereof and mixtures thereof.

Rigin-based tetrapeptides in accordance with the present invention are based on the structure Gly-Gln-Pro-Arg (Rigin) and include its analogs and derivatives thereof. Rigin is a preferred tetrapeptide. Analogs of the tetrapeptide rigin useful in accordance with the present invention include those in which one or more of the four amino acids are reorganized or rearranged within the sequence and/or where no more than two of the amino acids are substituted (e.g., Ala-Gln-Thr-Arg. More preferably, at least one of the amino acids within the sequence is Pro or Arg and most preferably the tetrapeptide includes both Pro and Arg although their order and position may vary. The amino acid substitutions can be from amongst any amino acid as defined herein. Particularly preferred rigin-based tetrapeptides include Xaa-Xbb-Arg-Xcc, Xaa-Xbb-Xcc-Pro, Xaa-Xbb-Pro-Arg, wherein Xaa-Xbb-Pro-Xcc, Xaa-Xbb-Xcc-Arg, Xaa, Xbb and Xcc may be the same or different and selected from the following Xaa is Gly or the amino acids that may be substituted therefore, Xbb is Gln or the amino acids that may be substituted therefore and Xcc may be Pro or Arg or the amino acids substituted therefore. The most preferable amino acids substituted for Gly include an aliphatic side chain such as, without limitation, beta-Ala, Ala, Val, Leu, Pro, Sarcosine (Sar) and Ile. The most preferable amino acids substituted for Gln include a side chain that includes an amine group that is predominantly uncharged at neutral pH (pH 6-7) such as, without limitation, Asn, Lys, Orn, 5-hydroxyproline, Citrulline and Canavanine. When Arg is substituted, it is preferably replaced with an amino acid having a side chain that includes, predominantly, a charged nitrogen at a pH of 6, such as, without limitation, Pro, Lys, His, Desmosine and Isodesmosine.

Derivatives are also considered to be encompassed by the term rigin-base tetrapeptides in accordance with the present invention, (and therefore also the more generic term tetrapeptides). Derivatives include derivatives of the substituted and rearranged rigin-based tetrapeptides described herein. These derivatives include, inter alia, acyl-derivatives, which are tetrapeptides substituted with one or more straight-chain or branched-chain, long or short chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO3H, SH or S-S.

ALAMCAT tetrapeptides are tetrapeptides which include at least one amino acid including an aliphatic group comprising side chain. These amino acids include, without limitation, Gly, beta-Ala, Ala, Val, Leu, Sarcosine (Sar) and Ile. These tetrapeptides also include at least one amino acid including at least one NH2 comprising side chain. These amino acids include a side chain that has an amine group that is predominantly uncharged at neutral pH (pH 6-7) such as, without limitation, Gln, Asn, Lys, Orn, 5-hydroxyproline, Citrulline and Canavanine. The ALAMCAT-tetrapeptides also include at least one amino acid having at least one side chain including at least one cationic amine (predominant species is charged such as NH3+ , NH2+ , etc.-basic amino acids which are positively charged at pH 6.0). These amino acids include, without limitation, Pro, Arg, Lys, His, Desmosine and Isodesmosine. The remaining amino acid can be any amino acid, but is preferably one comprising an alphatic group, pendant amino group or pendant cationic group. Derivatives are also considered to be encompassed by the term ALAMCAT-tetrapeptides in accordance with the present invention, (and therefore also the more generic term tetrapeptides). These derivatives include, inter alia, acyl-derivatives, which are tetrapeptides substituted with one or more straight-chain or branched-chain, substituted or unsubstituted long or short chain, saturated or unsaturated acyl group(s) having from 1 to 29 carbon atoms. The acyl groups which can be used are the same as those described for the rigin-based tetrapeptides.

Preferred embodiments include Peptide E, arg-ser-arg-lys, N-acyl-Gly-Gln-Pro-Arg peptides, most preferably N-palmitoyl-Gly-Gln-Pro-Arg.

Preferred commercially available sources of tetrapeptides include RIGIN, EYELISS, Haloxyl, and MATRIXYL 3000, which comprise between 50 to 500 ppm of palmitoyl-Gly-Gln-Pro-Arg, and other ingredients, such as peptides, chalcones and an excipient, commercially available from SEDERMA, France. Tego Pep 417 available from Evonik. These may be used to produce compositions of the present invention by adding thereto at least one tripeptide as described herein.

The tetrapeptides of the present invention are preferably used in amounts from 0.1 ppm (0.00001 % w/w also referred to herein as "weight percent", "weight %" or simply by weight) to 10,000 ppm (0.5% w/w), preferably from 0.5 ppm to 1000 ppm (0.05% w/w), and most preferably from 1 ppm to 500ppm by weight of the composition.

The combination of tripeptides and tetrapeptides, is particularly preferred. The preferred ratio of tetrapeptide to tripeptide, or indeed the ratio of molecules having four amino acids to those having three amino acids can range from 100:1 to 1:100; more preferably from 50:1 to 1:50, even more preferably from 30:1 to 1:30 and even more preferably between 10:1 to 1:10. Most preferably, the ratio of tetrapeptide to tripeptide ranges from between 3:1 to 1:3. These ratios are on a weight basis (% w/w-e.g. mg of pure peptide per Kilogram in the final formulation). In a particularly preferred embodiment, the amount of tripeptide used is greater than the amount of tetrapeptide used when considered in terms of their amounts in parts per million, again based on overall weight of the composition. In a particularly preferred embodiment, the composition of the present invention comprise a tetrapeptide of the sequence Gly-Gln-Pro-Arg, its analogs and derivatives in combination with one or more tripeptide of the sequences Gly-His-Lys, its analogs and derivatives.

Pentapeptides

The compositions of the present invention may optionally comprise a pentapeptide, derivatives of pentapeptides, and mixtures thereof. As used herein, "pentapeptides" refers to both the naturally occurring pentapeptides and synthesized pentapeptides. Also useful herein are naturally occurring and commercially available compositions that comprise pentapeptides. Suitable pentapeptides are those selected from the group consisting of pentapeptide1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof and mixtures thereof.

Suitable pentapeptides for use herein are the pentapeptide, lys-thr-thr-lys-ser, Arg-asp-lys-tyr-val (pentapeptide -1) and derivatives thereof. A preferred commercially available pentapeptide derivative-comprising composition is Matrixyl which comprises 100 ppm of palmitoyl-lys-thr-thr-lys-ser and is commercially available from Sederma, France.

The pentapeptides and/or pentapeptide derivatives where present are preferably included in composition at amounts of from 0.01% to 20%, more preferably from 0.05% to 15%, and even more preferably from 0.1% to 10%, by weight of the composition.

Matrix metalloproteinase inhibitors (MMPi)

The term "matrix metalloproteinase inhibitor" relates to all molecule and/or plant or bacterial extracts having an inhibitory activity on at least one of the matrix metalloproteinases expressed or synthetized by or in the skin. The family of the matrix metalloproteinases is formed of several well-defined groups on the basis of their resemblance regarding structure and substrate specificity (Woessner J. F., Faseb Journal, vol. 5,1991, 2145). Among these groups, there are collagenases able to degrade fibrillar collagens (MMP-1 or interstitial collagenase, MMP-8 or neutrophil collagenase, MMP- 13 or collagenase 3, MMP-18 or collagenase 4), gelatinases degrading type IV collagen or other denatured collagen form (MMP-2 or A gelatinase (72 kDa), MMP-9 or B gelatinase (92 kDa)), stromelysins (MMP-3 or stromelysin 1, MMP- 10 or stromelysin 2, MMP-11 or stromelysin 3) whose broad spectrum of activity targets proteins of the extracellular matrix such as glycoproteins (fibronectin, laminin), proteoglycanes etc., matrilysin (MMP-7), metalloelastase (MMP- 12) or metalloproteinases (MMP- 14, MMP- 15, MMP- 16 and MMP- 17). Metalloproteinases (MMPs) are proteases that use a metal, (mostly zinc) coordinated to 3 cystein residues and to a methionine in their active site , that degrade macromolecular components of the extracellular matrix and of basal layers at neutral pH (collagen, elastin, etc ...). This group of enzymes is inactivated by metal chelators. The principal activity regulators of MMPs are the tissue inhibitors of metalloproteinases or TIMPs such TIMP-l, TIMP-2, TIMP-3 and TIMP-4 (Woessner J. F., Faseb Journal, 1991). Furthermore, the MMPs expression is also regulated by growth factors, cytokins, oncogens products (ras, jun), or also matrix constituents.

The term "matrix metalloproteinase inhibitors "according to the present invention means all molecules able to reduce the MMPs activity regarding the gene expression (transcription and translation) or regarding the activation of the zymogen form of MMPs, or else regarding the local control of active forms. Furthermore, the metalloproteinase inhibitors according to the present invention can also be MMP-1 inhibitors of natural or synthetic origin. The terms "natural origin" or "synthetic origin" mean both a metalloproteinase inhibitor at a pure state or in solution at different concentrations, but natural origin termed inhibitors are obtained by different extraction methods from a natural element (for example lycopene from a tomato) whereas the inhibitors of synthetic origin are all obtained via chemical synthesis

Compositions of the present invention preferably comprise an MMPi. Preferred MMPi are selected from the group consisting of retinoid, N-acetyl cysteine, glutathione, 2-furildioxime, vitamin C, hydrolysed rice protein, alfalfa extract, white lupin, zizyphus jujube extract, kudzu extract, vitis vinifera extract, Oenothera biennis extract Anogeissus leiocarpus extract and mixtures thereof.

Where present, MMPi are present at a level of from 0.01% to 10%, more preferably 0.1% to 5% and most preferably from 1% to 2.5% by weight of the composition.

Skin Conditioning Agent

The composition of the present invention may optionally comprise a skin conditioning agent. Said skin conditioning agents may preferably be selected from the group consisting of humectants, emollients, moisturisers, or mixtures thereof. Where present, they are preferably present at a level of from 0.01% to 20%, more preferably from 0.1% to 10%, most preferably from 0.5% to 7% by weight of the composition. Preferred skin conditioning agents are selected from the group consisting of guanidine, urea, glycolic acid and glycolate salts, salicylic acid, lactic acid and lactate salts, aloe vera, shea butter, polyhydroxy alcohols, such as sorbitol, mannitol, xylitol, erythritol, glycerol, hexanetriol, butanitriol, (di) propylene glycol, butylene glycol, hexylene glycol, polyethylene glycol, sugars (e.g. fructose, glucose, xylose, honey, mannose, xylose), gluconodeltalactone, and starches and their derivatives, pyrrolidone, carboxylic acid, hyaluronic acid and salts thereof, lactamide monoethanolamine, acetamide monoethanolamine, panthenol, allantoin and mixtures thereof. More preferably said skin conditioning agent is selected from glycerine, arabinoglactan, butylene glycol, hyaluronic acid, shea butter, propylene glycol, ethylhexyl glycerine, hyaluronate and mixtures thereof.

Salicylic Acid Compound

The compositions of the present invention may comprise a salicylic acid compound, its esters, its salts, or combinations thereof. In one embodiment of the compositions of the present invention, the salicylic acid compound preferably comprises from 0.0001% to 25%, more preferably from 0.001% to 15%, even more preferably from 0.01% to 10%, still more preferably from 0.1% to 5%, and even more preferably from 0.01% to 2%, by weight of the composition, of salicylic acid.

Sunscreen

The composition of the present invention may optionally, but preferably comprise a sunscreen component. The sunscreen may comprise organic or inorganic sun filters or a combination of the two. Suitable inorganic sunfilters include those selected from the group consisting of microfine titanium dioxide, microfine zinc oxide, boron nitride and mixtures therof.

Suitable organic sunscreens include those selected from the group consisting of : a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'-(2ethylhexanoyl)-glycerin, c) benzophenones (for example oxybenzone), d) dibenzoylmethanes such as 4- (tert-butyl)-4'-methoxydibenzoylmethane, e) 2-phenylbenzimidazole-5 sulfonic acid and its salts, f) alkyl-ss, ss-diphenylacrylates for example alkyl $\alpha$-cyano-ss, ss-diphenylacrylates such as octocrylene, g) triazines such as 2,4,6-trianilino- (p-carbo-2-ethyl-hexyl-1-oxi)-1,3,5 triazine, h) camphor derivatives such as methylbenzylidene camphor and i) mixtures thereof. Other preferred sunscreen ingredients include those selected from the group consisting of homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15 and mixtures thereof. A sunscreening agent is optionally present in an amount from 0.1 to 10% by weight of the composition.

Other Optional Ingredients

The compositions of the present invention may also optionally comprise one or more of the following optional ingredients. Preservatives may be added to the composition such as 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, isothiazolone, sodium propyl paraben and mixtures thereof, suitably in an amount of from 0.01% to 10% by weight of the composition.

Thickeners, viscosity modifying agents and/or gelling agents may be added to the composition, such as acrylic acid polymers e. g. available commercially under the trade name Carbopol or Ultrez (Lubrizol) or modified cellloses e. g. hydroxyethylcellulose available commercially under the trade name Natrosol (Hercules) or hydroxypropylmethyl cellulose, amine oxides, block polymers of ethylene oxide and propylene oxide (for example, those available from BASF Wyandotte under the trade name"Pluronic"RTM), PVM, MA, or a decadiene crosspolymer (available under the trade name Stabilez 60), ethoxylated fatty alcohols, salt (magnesium chloride, sodium chloride), Aristoflex AVC (Clariant), phthalic acid amide, xanthan gum, sodium polyacrylate, polyvinyl alcools, fatty alcools and alkyl galactmanans available under the trade name N-Hance from Hercules, suitably in an amount of from 0.5% to 10% by weight of the composition.

Sequestering agents may be added to the composition, such as ethylenediamine tetraacetic acid and salts thereof, preferably in an amount of from 0.005% to 0.5% by weight of the composition. The composition may also include waxes such as cocoa butter suitably in an amount of from 1% to 99% by weight of the composition. The composition may also comprise suitable, cosmetically acceptable diluents, carriers and/or propellants such as dimethyl ether. The composition may also include pearlising agents such as stearic monoethanolamide and/or mica, suitably in an amount of from 0.01% to 10% by weight of the composition. Perfumes may be added suitably in an amount of from 0.01% to 2% by weight of the composition, as may water soluble dyes such as tartrazine, suitably in an amount of from a trace amount (such as 1 x 10-5 %) to 0.1 % by weight of the composition.

The composition may also include pH adjusting agents such as sodium hydroxide, aminomethyl propanol, triethanolamine, suitably in an amount of from 0.01 % to 10% by weight of the composition. The composition may be buffered by means well known in the art, for example by use of buffer systems comprising succinic acid, citric acid, lactic acid, and acceptable salts thereof, phosphoric acid, mono-or disodium phosphate and sodium carbonate. Suitably, the composition may have a pH between 3 and 10, preferably between 4 and 8.

Total Antioxidant Capacity (TAC) Assay

Antioxidant activity of the plant phenolic agents is measured using Total Antioxidant Capacity (TAC) assay. The present assay describes the method used to assess the capacity of putative antioxidants to reduce copper (II) to copper (I), utilising the electron transfer (and thus reducing) capacity of antioxidants to cause this chemical reaction and associated colour change, which can be quantified.

Materials used

- Oxiselect Total Antioxidant Capacity (TAC) assay kit, Cell Biolabs (STA-360)
- Methanol
- Active ingredients for complexes
- $\alpha$-tocopherol (sigma-Aldrich)
- 1.5ml eppendorf tubes
- Pipettes and tips
- 96 well plates

Plate reader

Preparation of reagents

Before the assay, reagents from the assay kit were freshly prepared as follows:

1X Reaction Buffer: The Reaction Buffer was diluted 1:100 with methanol then mixed thoroughly.
1X Copper Ion Reagent: The Copper Ion Reagent was diluted 1:100 with methanol then mixed thoroughly.
1X Stop Solution: The Stop Solution was diluted 1:10 with methanol then mixed thoroughly.

Preparation of samples

Samples were initially made up by dissolving the active ingredients in methanol to the following concentrations (all percentages shown are in weight per volume);
Comparative Complex 1 = mulberry 0.2% (w/v), ginseng 0.1% (w/v), ascorbyl glucoside 0.05% (w/v);
Comparative Complex 2 = mulberry 0.2%(w/v), ginseng 0.2%(w/v), ascorbyl glucoside 0.1%(w/v), tocopherol acetate 0.1%(w/v);
Present invention= mulberry 0.2%(w/v), ginseng 0.2%(w/v), ascorbyl glucoside 0.1%(w/v), tocopherol acetate 0.1%(w/v), dimethyl methoxy chromanol 0.015%(w/v)

Complexes were made up in a total volume of 1ml methanol, in 1.5ml eppendorf tubes. The complexes as listed above were then further diluted 1:10 in methanol (by adding 100$\mu$l of the original sample to 900$\mu$l methanol in a separate 1.5ml eppendorf tube), before being run in the assay. This was to keep the data generated within the range of the assay kit used, as without further dilution the antioxidants were too powerful for the assay kit and above the linear limit of the assay.

Preparation of controls

An α-tocopherol standard curve was run alongside the test samples to act as a positive control. This standard curve was run between the range of 1mM and 0.125mM, and run at the following increments: 0.125mM, 0.25mM, 0.5mM and 1mM. As with the test samples, α-tocopherol controls were dissolved in 1ml methanol in 1.5ml eppendorf tubes.

Assay protocol

All samples were run in triplicate, so n=3 for all controls and complexes.

The assay was run as follows:

1. 20μl of each tocopherol positive control or sample were added to individual wells of a 96 well plate, in triplicate. Methanol was added to a further 3 wells to act as a negative control.
2. 180μl of the pre-prepared 1X Reaction Buffer was added to each well using a multichannel pipette.
3. An initial absorbance of the plate was read at 490nm.
4. To initiate the reaction, 50μl of the pre-prepared 1X Copper Ion Reagent was added to each well. This was then incubated for 5 minutes on an orbital shaker at room temperature.
5. 50μl of the pre-prepared 1X Stop Solution was then added to each well to terminate the reaction.
6. The plate was then read again at 490nm to obtain a reaction read.

Analysis

For each well, the initial absorbance reading was subtracted from the final reaction read. This removed any background from each individual well. As all controls and samples were run in triplicate, the mean of each sample and control was then calculated. The mean negative control reading was then subtracted from each sample and α-tocopherol positive control to give a final absorbance reading for each sample tested.

As absorbance directly relates to the amount of copper II reduced in the assay, this absorbance relates to the antioxidant ability of the sample.

The positive control standard curve was used both to test the accuracy of the assay, and as a comparison of efficacy for the complexes tested. As 0.25mM was the central point in the assay with regard to the samples tested, this was used as it was the most useful comparator for samples.

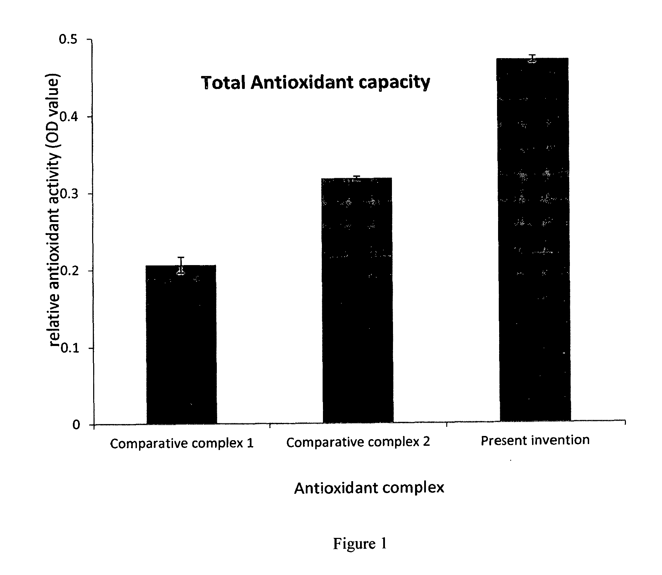

The results of the present assay are shown in Figure 1 and show the synergistic increase in antioxidant activity in the complex of the present invention comprising plant polyphenolic agent, vitamin E and dimethyl methoxy chromanol, as compared to prior comparative complexes 1 and 2.

Lipid peroxidation (LPO) Assay

Lipids are present in the cell membrane bilayer. Peroxidation of lipids compromises the integrity of skin cells and attenuates the ageing process resulting in premature ageing. The LPO assay is designed to determine the level of LPO damage in the skin with and without external stresses such as UV exposure; and investigating whether early defence complex can protect against the UV induced LPO damage.

Materials and Methods

K-assay LPO-CC004 lipid peroxidation kit (Kamiya biomedical company) -500 assays containing -

| | |
|---|---|
| Reagent 1 (liquid): | Enzyme reagent (ascorbic oxidase, lipoprotein lipase, stabilizer, goods buffer, surfactants) |
| Reagent 2A: | Lyophilised chromogen reagent (MCDP for 110ml reagent 2B) |
| Reagent 2B: | Buffer solution (liquid) (goods buffer, hemoglobin, surfactants) |
| Calibrator (liquid): | (50 nmol/ml cumene hyderoperoxide) |

D-squames (CuDerm Corp. Dalla, USA)

Linoleic acid (L.A)

Methanol (MeOH)

Deionised water (dH$_2$O)

96 well flat bottom clear plate

(continued)

Plate reader

**Sample preparations:**

Unless otherwise stated all the samples were prepared weight per volume in methanol.

1: 2% Linoleic acid

2: 2% Linoleic acid + 1% trolox

3: 2% Linoleic acid + 0.1% Lipochroman 6 (Lipoc)

4: 2% linoleic acid + 0.001% Lipochroman 6 (lipoc)

5: comparative complex 3, prepared in Methanol comprising

- Mulberry 0.2%
- Ginseng 0.2%
- Tocopherol acetate 0.1%
- Ascorbyl glucoside 0.3%

6: present invention, prepared in Methanol

- Mulberry 0.2%
- Ginseng 0.2%
- Tocopherol acetate 0.1%
- Lipochroman 0.015%
- Ascorbyl glucoside 0.3%

Assay Protocol:

1. 20 $\mu$l of each above sample was added to 96 well plate in triplicates. The plate was sealed with UV transparent lid.
2. The samples were irradiated under solar simulator at a UV dose of 36 X 10$^4$ J/m$^2$.
3. A two-point calibration curve was made using 20 $\mu$l of 50 nmol/ml cumene hyderoperoxide.
1. After irradiation, following LPO-CC kit instructions, 80 $\mu$l of enzyme complex was added to above samples.
2. The plate was incubated for 5 min at 30°C, to allow the blocking of any non-specific lipid peroxide production.
3. 160 $\mu$l of chromogen reagent is added. The plate is incubated for 10 min at 30°C for measuring the production of lipid peroxidation.
4. Absorbance was read at 675 nm.

**Data Analysis:**

For each well, the methanol background reading was subtracted from the absorbance reading. This removed any background from each individual well. As samples were run in triplicate, the mean of each sample was then calculated.

The two-point positive control standard curve was used both to test the accuracy of the assay, and as a comparison of efficacy for the complexes tested.

For method 3, LPO values were determined from using the following equation as per kit instructions:

$$\text{LPO value in nmol/ml} = \frac{\text{Absorbance (sample) - Absorbance (methanol) X 50}}{\text{Absorbance (calibrator) - Absorbance (methanol)}}$$

All the tape strips were weighed before and after stripping, obtaining the weight of stratum corneum removed (Cristina et al., 2009). The LPO values were then normalised against the weights of tape strips to accurately measure the lipid peroxidation against lipid content on present on each tape strip.

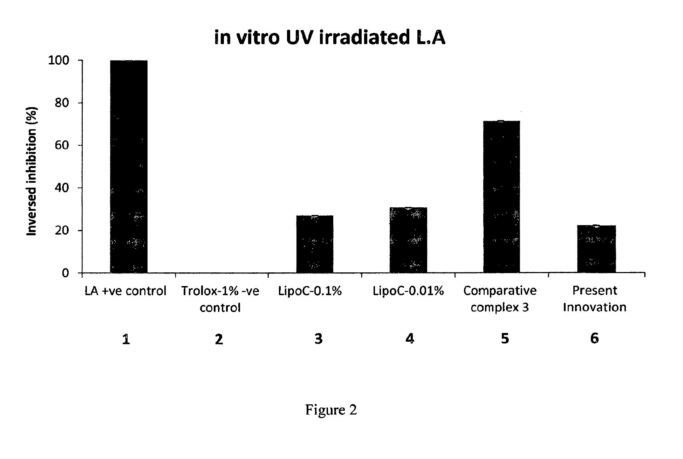

The results of the above experiments are represented in figure 2. In Figure 2, column 1, titled LA+ve, represents a positive control, showing 100% oxidation, the worst case scenario event. Column 2, titled Trolox 1%, is a negative control, showing very low oxidation, and maximum oxidative inhibition. Column 3, LipoC at 0.1% and column 4, lipoc at 0.01%, show the extent of oxidation at different levels of dimethyl methoxy chromanol (LipoC). Column 5, comparative complex 3, shows the level of oxidation seen in a complex not comprising dimethyl methoxy chromanol. Column 6 shows the effect on oxidation of the complex of the present invention. As can be seen, not only does the complex of the present invention perform better than comparative complex 3 in inihibiting oxidation, but it also shows a synergy within the complex. The complex of the present invention, comprising 0.015% dimethyl methoxy chromanol, performs better than the experiments comprising equivalent amount of dimethyl methoxy chromanol (0.01%) and that comprising ten times the amount (0.1%) dimethyl methoxy chromanol).

Examples

The below represent non-binding examples of the present invention. The amount of incorporation is represented as a percentage by weight of the composition.

|  | 1 | 2 | 3 | 4 | 5 | 6 |
|---|---|---|---|---|---|---|
| C12-15 alkyl benzoate | 5 | 8 | 5 | 10 | 5 | 10 |
| Cetearyl glucoside, | 1.4 | 1.2 | 1.5 | 1.4 | 1.4 | 1.4 |
| Potassium cetyl phosphate | 0.4 | 0.2 | 0.4 | 0.5 | 0.4 | 0.5 |
| Butylene glycol | 5 | 5 | 6 | 3 | 5 | 3 |
| Potassium hydroxide | 0.2 | 0.2 | 0.2 | 0.2 | 0.2 | 0.2 |
| Carbomer | 0.9 | 0.9 | 0.9 | 0.9 | 0.9 | 0.9 |
| Octocrylene | 4 | 4 | 4 | 4 | 4 | 4 |
| Ethylhexyl salicylate | 1 | 1 | 1 | 1 | 1 | 1 |
| Butyl methoxydibenzoylmethane | 3.5 | 3.5 | 3.5 | 3.5 | 3.5 | 3.5 |
| Bis-ethylhexyloxyphenol methoxyphenyl triazine | 0.5 | 0.5 | 0.5 | 0.5 | 0.5 | 0.5 |
| Diethylhexyl butamido triazone | 0.1 | 0.1 | 0.1 | 0.1 | 0.1 | 0.1 |
| Tocopheryl acetate | 0.2 | 0.1 | 0.5 | 0.2 | 1 | 0.1 |
| Ascorbyl glucoside | 0.1 | 0.1 | 0.2 | 0.3 | 0.1 | 2 |
| Morus alba leaf extract | 0.2 | 0.2 | 0.25 | 0.2 | 0.1 | 0.05 |
| Panax ginseng root extract | 2 | 0.2 | 0.4 | 0.2 | 0.25 | 1 |
| Gingko Biloba |  | 0.2 | 1 |  |  |  |
| Green Tea | 0.1 |  |  |  |  |  |
| French Pine Bark |  | 1 |  | 0.2 |  |  |
| Oreganum |  |  |  |  |  | 0.1 |
| Emblica officianalis |  |  |  |  |  | 0.2 |
| Green Coffee |  |  |  |  | 0.2 |  |
| Rosemary | 0.1 |  |  |  |  |  |
| Dimethylmethoxy chromanol | 0.015 | 0.1 | 0.5 | 0.001 | 0.015 | 1 |
| Hydrolyzed rice protein | 0.25 | 0.25 | 0.5 | 0.5 | 2 | 1 |
| Medicago sativa (Alfalfa) extract | 0.25 | 0.25 | 0.5 | 1 | 0.01 | 1 |
| Water | To 100 | To 100 | To 100 | To 100 | To 100 | To 100 |

EP 3 069 763 A1

**Claims**

1. A cosmetic liquid composition comprising an antioxidant complex, said complex comprising

    a) at least one antioxidant polyphenolic agent
    b) at least one vitamin E or a vitamin E derivative; and
    c) a chromane antioxidant.

2. A composition according to claim 1 wherein the antioxidant polyphenolic agent is a plant extract selected form the group consisting of mulberry, ginseng, grape, oregano, grape, sage, sunflower, maritime pine bark, rosemary, marjoram, crocus, french saffron, wild carrot, hop, coffee ,green coffee, witch hazel, oak, camellia, red clover, flax, ginger, magnolia, edelweiss, burdock, emblica, gingko, lemon, blueberry, apple, cherry, birch, moringa, thyme, cornflower, geranium, french saffron, white lotus, marshmallow, bilberry, cranberry extract, pomegranate nectar, polygonum, green tea, white tea, soy and mixtures thereof.

3. A composition according to the preceding claim comprising at least two antioxidant polyphenolic agents.

4. A composition according to any preceding claim wherein at least one antioxidant polypenolic agent is a plant extracts selected from the group consisting of mulberry, ginseng, grape, oregano, grape, sage, sunflower, maritime pine bark, rosemary, marjoram, crocus, french saffron, wild carrot, hop, coffee ,green coffee, witch hazel, oak, camellia, red clover, flax, ginger, magnolia, edelweiss, burdock and mixtures thereof.

5. A composition according to any preceding claim comprising from 0.005% to 10% by weight, preferably 0.01% to 5%, most preferably 0.01% to 3.5%polyphenolic agent.by weight of the composition.

6. A composition according to any preceding claim wherein the vitamin E or vitamin E derivative is selected from the group consisting of alpha tocopherol, beta tocopherol, gamma tocopherol, delta tocopherol, epsilon tocopherol, zeta tocopherol, eta tocopherol, tocopheryl acetate, tocopheryl succinate, tocopheryl benzoate, tocopheryl propionate, tocopheryl sorbate, tocopheryl oleate, tocopheryl orotate, tocopheryl linoleate, tocopheryl nicotinate, 2-ethylhexanoate ester and mixtures thereof.

7. A composition according to any preceding claim wherein the vitamin E or vitamin E derivative is present at a level of from 0.01 % to 5%.

8. A composition according to any preceding claim wherein the chromane antioxidant is selected from the group consisting of dimethyl methoxy chromanol, tetramethyl methoxy chromanol, pentamethyl chromanol, dimethyl methoxy chomanyl palmitate, dialkyl methoxy chomanyl myristate, dimethyl methoxy chromanyl stearate, dimethyl methoxy chomanyl oleate, dimethyl methoxy chomanyl linoleate and mixtures thereof.

9. A composition according to any preceding claim wherein the chromane antioxidant is dimethyl methoxy chromanol.

10. A composition according to any preceding claim wherein the chromane antioxidant is present at a level of from 0.001% to 5% by weight of the composition.

11. A composition according to any preceding claim additionally comprising a vitamin c species selected from the group consisting of ascorbic acid its salts, esters, glucosides and glucosamines.

12. A composition according to claim 11 wherein the vitamin C species is selected from the group consisting of ascorbic acid, ascorbyl glucoside, ascorbyl palmitate, magnesium ascorbyl phosphate, sodium ascorbyl phosphate and mixtures thereof.

13. A composition according to any preceding claim additionally comprising a matrix metalloproteinase inhibitor (MMPi).

14. A composition according to claim 13 wherein the MMPi is selected from the group consisting of retinoid, N-acetyl cysteine, glutathione, 2-furildioxime, vitamin C, hydrolysed rice protein, alfalfa extract, white lupin, zizyphus jujube extract, kudzu extract, vitis vinifera extract, Oenothera biennis extract Anogeissus leiocarpus extract and mixtures thereof.

14

15. A composition according to any preceding claim additionally comprising an organic and/or inorganic UVA and/or UVB blocking agent.

16. A composition according to claim 15 wherein inorganic UV blocking agents are selected from the group consisting of titanium dioxide, zinc oxide, boron nitride and mixtures thereof.

17. A composition according either of claims 15 and 16 wherein the composition comprises an organic UV blocking agent selected from the group consisting of: a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4- (tert-butyl)-4'-methoxydibenzoylmethane, e) 2-phenylbenzimidazole-5 sulfonic acid and its salts, f) alkyl-ss, ss-diphenylacrylates for example alkyl a-cyanoss, ss-diphenylacrylates such as octocrylene, g) triazines such as 2,4,6-trianilino- (p-carbo-2-ethyl-hexyl-1-oxi)-1, 3,5 triazine, h) camphor derivatives such as methylbenzylidene camphor and i) mixtures thereof.

18. A method of cosmetically treating skin wherein a composition according to any preceding claim is applied to the skin.

Figure 1

Figure 2

Europäisches
Patentamt

European
Patent Office

Office européen
des brevets

# EUROPEAN SEARCH REPORT

Application Number

EP 15 00 0770

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/315995 A1 (DREHER FRANK [US]) 23 October 2014 (2014-10-23) * paragraphs [0008] - [0009], [0017] - [0018], [0022] - [0025], [0078] - [0093], [0117], [0143], [0183] - [0185]; examples 1, 2a, 2b * * Product A; table 3 * * claims 1-2, 4,9-10, 14-16 * | 1-18 | INV. A61Q17/04 A61K8/97 A61K8/49 A61K8/67 A61Q19/08 |
| X | EP 2 253 305 A2 (HENKEL AG & CO KGAA [DE]) 24 November 2010 (2010-11-24) * page 26 - page 27; example 16 * | 1-18 | |
| X | DE 10 2008 053273 A1 (HENKEL AG & CO KGAA [DE]) 29 April 2010 (2010-04-29) * column 87 * | 1-18 | |
| X | WO 2013/149323 A1 (NTEGRITY [CA]) 10 October 2013 (2013-10-10) * example 8 * | 1-18 | |
| X | US 2006/198800 A1 (DILALLO NATALIE [US] ET AL) 7 September 2006 (2006-09-07) * example 20 * | 1-18 | TECHNICAL FIELDS SEARCHED (IPC) A61Q A61K |
| X | EP 2 266 527 A2 (HENKEL AG & CO KGAA [DE]) 29 December 2010 (2010-12-29) * Table linking pages 5-6 * * paragraphs [0054], [0091] - [0095] * * Table linking pages 28-29; claim 1 * | 1-18 | |
| X | WO 2013/066623 A1 (ARSHED KHALEEQ [US]; VASKIN LLC [US]) 10 May 2013 (2013-05-10) * page 15, lines 1-12 * * page 39 - page 45; examples 1-4 * * claims 14-19 * | 1-18 | |

-/--

The present search report has been drawn up for all claims

| Place of search | Date of completion of the search | Examiner |
|---|---|---|
| Munich | 16 October 2015 | Paul Soto, Raquel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 15 00 0770

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2009/044190 A1 (BOOTS CO PLC [GB]; BARTON STEPHEN PETER [GB]; JOHNSON MARK [GB]; TOMLI) 9 April 2009 (2009-04-09) * the whole document * | 1-18 | |
| A,D | WO 01/17495 A1 (BOOTS CO PLC [GB]; PYKETT MELANIE ANN [GB]; CRAIG AILSA HELEN [GB]; GA) 15 March 2001 (2001-03-15) * the whole document * | 1-18 | |

TECHNICAL FIELDS
SEARCHED (IPC)

The present search report has been drawn up for all claims

| Place of search | Date of completion of the search | Examiner |
|---|---|---|
| Munich | 16 October 2015 | Paul Soto, Raquel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 00 0770

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2015

| Patent document cited in search report | | Publication date | Patent family member(s) | | Publication date |
|---|---|---|---|---|---|
| US 2014315995 | A1 | 23-10-2014 | US 2014315995 A1<br>WO 2014188276 A2 | | 23-10-2014<br>27-11-2014 |
| EP 2253305 | A2 | 24-11-2010 | DE 102009026414 A1<br>EP 2253305 A2 | | 25-11-2010<br>24-11-2010 |
| DE 102008053273 | A1 | 29-04-2010 | DE 102008053273 A1<br>WO 2010049389 A1 | | 29-04-2010<br>06-05-2010 |
| WO 2013149323 | A1 | 10-10-2013 | NONE | | |
| US 2006198800 | A1 | 07-09-2006 | US 2006198800 A1<br>WO 2007102957 A2 | | 07-09-2006<br>13-09-2007 |
| EP 2266527 | A2 | 29-12-2010 | DE 102009027105 A1<br>EP 2266527 A2 | | 30-12-2010<br>29-12-2010 |
| WO 2013066623 | A1 | 10-05-2013 | US 2013195925 A1<br>WO 2013066623 A1 | | 01-08-2013<br>10-05-2013 |
| WO 2009044190 | A1 | 09-04-2009 | EP 2197413 A1<br>ES 2523305 T3<br>US 2010221202 A1<br>WO 2009044190 A1 | | 23-06-2010<br>24-11-2014<br>02-09-2010<br>09-04-2009 |
| WO 0117495 | A1 | 15-03-2001 | AT 310495 T<br>AU 7001500 A<br>DE 60024290 D1<br>DE 60024290 T2<br>EP 1217983 A1<br>JP 2003508477 A<br>US 7195787 B1<br>WO 0117495 A1 | | 15-12-2005<br>10-04-2001<br>29-12-2005<br>21-09-2006<br>03-07-2002<br>04-03-2003<br>27-03-2007<br>15-03-2001 |

EPO FORM P0459

For more details about this annex : see Official Journal of the European Patent Office, No. 12/82

**EP 3 069 763 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1217983 A
- EP 2197413 A
- US 5412004 A
- US 5837793 A
- US 5811487 A
- US 4970252 A
- US 5760116 A
- US 5654362 A

**Non-patent literature cited in the description**

- **WOESSNER J. F.** *Faseb Journal,* 1991, vol. 5, 2145
- **WOESSNER J. F.** *Faseb Journal*